# EUROPEAN PATENT APPLICATION

(11) **EP 4 026 483 A1**
(43) Date of publication of application: **13.07.2022**
(21) Application number: 21151144.9
(22) Date of filing: 12.01.2021
(51) Int. Cl.: A61B 5/00, A61B 5/01, A61B 5/145, A61N 1/32, A61N 1/36, G08B 21/02

(54) **STIMULATION OF SWEAT GENERATION**

(71) Applicant: Koninklijke Philips N.V., 5656 AG Eindhoven (NL)
(72) Inventor: PELSSERS, Eduard Gerard Marie, 5656 AE Eindhoven (NL); VAN LIESHOUT, Ron Martinus Laurentius, 5656 AE Eindhoven (NL); DELLIMORE, Kiran Hamilton J., 5656 AE Eindhoven (NL); GERHARDT, Lutz Christian, 5656 AE Eindhoven (NL); JOHNSON, Mark Thomas, 5656 AE Eindhoven (NL); HUIJBREGTS, Laurentia Johanna, 5656 AE Eindhoven (NL)
(74) Representative: Philips Intellectual Property & Standards

(57) **Abstract**

According to an aspect, there is provided a system (10) comprising a sweat stimulation device (100) for stimulating sweat generation from sweat glands of a subject, the device comprising: a stimulation element (102) configured to provide modulated stimulation to the sweat glands through the skin of the subject; and a sensor (104) for measuring data indicative of a characteristic of sweat generated by the sweat glands in response to the stimulation; and a processor (110) configured to receive the measured data from the sensor; compare the measured data with a threshold value; and responsive to determining that the measured data meets or exceeds the threshold value, generate an alert signal.

## Description

### FIELD OF THE INVENTION

The invention relates to a method for stimulating sweat generation and, more particularly, to a method for stimulating sweat generation for measuring a characteristic of sweat.

### BACKGROUND OF THE INVENTION

Sweat is a non-obtrusively accessible bio-fluid containing physiologically and metabolically rich information. Sweat contains biomarkers that can be used to indicate the health and well-being of a subject whose sweat is being monitored, such as monitoring dehydration, stress, sleep, children's health and in perioperative monitoring.

The development of reliable sweat sensing has been limited due to a number of factors including, among others, a subject's ability to produce enough sweat for analysis. Hence, a local sweat stimulation method is in demand for sufficient sweat to enable measurements. There are various methods to stimulate sweat production, both pharmaceutical and non-pharmaceutical. However, the problems are that these methods are laborious, only suitable in laboratories and do not work for regular or semi-continuous measurements with patients or in lifestyle applications. Especially continuous stimulation induces adaptation causing the so-called die-away effect, reducing sweat rate.

There is therefore a desire for a method and system capable of stimulating sweat generation from a subject in a way which addresses at least one of these issues.

### SUMMARY OF THE INVENTION

Existing techniques are capable of increasing the amount of sweat generated by a person, but do not address many of the known issues, such as those discussed above. Thus, existing sweat sensing devices tend to be used only on people who generate a sufficient amount of sweat (e.g. athletes). The inventors of the present disclosure have recognised that various sweat stimulation techniques may be implemented which cause a subject to generate a sufficient amount of sweat for analysis, but which do not result in the person's body adapting to the techniques, such that the sweat rate reduces. Specifically, techniques disclosed herein involve the use of a sweat stimulation mechanism, which is modulated in some way, such that the person is not subjected to the stimulation technique for a prolonged, continuous period of time. Rather, modulating the stimulation involves varying the stimulation such that the person's body continues to respond and generate sweat, without adapting to the stimulation.

According to a first specific aspect, there is provided a system comprising a sweat stimulation device for stimulating sweat generation from sweat glands of a subject, the device comprising a stimulation element configured to provide modulated stimulation to the sweat glands through the skin of the subject; and a sensor for measuring data indicative of a characteristic of sweat generated by the sweat glands in response to the stimulation; and a processor configured to receive the measured data from the sensor; compare the measured data with a threshold value; and responsive to determining that the measured data meets or exceeds the threshold value, generate an alert signal.
The stimulation element may comprise at least one of: a heating element operative to apply a modulated stimulation comprising a variation in temperature; and an electrical element operative to apply a modulated stimulation comprising a variation in electrical current. The sweat stimulation device may comprise a channel configured to transport sweat from skin of the subject to the sensor; a reservoir for containing a fluid, the reservoir in fluidic communication with the channel; and a reservoir heater to heat fluid contained within the reservoir, thereby to increase the humidity in the channel.
In some embodiments, the sweat stimulation device may further comprise at least one of: a temperature sensor to measure temperature data indicative of a temperature of a region of the skin of the subject around the stimulation element; and a skin conductance sensor to measure a response signal indicative of skin conductance of the subject.

According to a second specific aspect, there is provided a computer-implemented method of stimulating sweat generation from sweat glands of a subject, the method comprising: operating a stimulation element to provide modulated stimulation to the sweat glands through skin of the subject, the modulated stimulation comprising at least one period of increased stimulation and at least one period of reduced stimulation; receiving, from a sweat sensor, data indicative of a characteristic of sweat generated by the sweat glands in response to the stimulation; comparing the received data with a threshold value; and responsive to determining that the received data meets or exceeds the threshold value, generating an alert signal.

The stimulation element may, in some embodiments, comprise a heating element. The modulated stimulation may comprise a variation in temperature of the heating element, such that the at least one period of increased stimulation comprises an increase in temperature of the heating element and the at least one period of reduced stimulation comprises a decrease in temperature of the heating element.

The method may further comprise receiving, from a temperature sensor, temperature data indicative of a temperature of a region of the skin of the subject around the heating element; and
adjusting an operating parameter of the heating element based on the received temperature data.

In other embodiments, the stimulation element may comprise an electrical element operative to conduct an electrical current through the skin. The modulated stimulation may comprise a series of electrical pulses, such that the at least one period of increased stimulation comprises an increase in electrical current and the at least one period of reduced stimulation comprises a decrease in electrical current. Use of an electrical element may be advantageous as it does not increase the temperature of the subject's skin and, therefore, may provide little or no discomfort to the subject. Furthermore, incorporating an electrical element (e.g. a wire) into the sweat stimulation device may be easier in manufacturing terms than fluidic channels. With electrical pulses, the application can be immediate, meaning that the application of electrical stimulation can be very time specific.

In some embodiments, the method may further comprise receiving, from a skin conductance sensor, a response signal indicative of skin conductance of the subject; and adjusting an operating parameter of the electrical element based on the received stimulus response signal.

The method may further comprise receiving, from a skin conductance sensor, a response signal indicative of skin conductance of the subject; and responsive to determining that the received response signal meets a defined condition, activating the sweat sensor. The method, in some embodiments, may further comprise operating the stimulation element to modify, during the at least one period of increased stimulation, at least one of a duration, an amplitude, a frequency and a duty cycle of the modulated stimulation. In some embodiments, the method may comprise determining, using the sweat sensor, an indication of an amount of lactate generated by the sweat glands; and comparing the determined amount of lactate to a lactate threshold value. Responsive to determining that the amount of lactate meets or exceeds the lactate threshold value, the alert signal may comprise an alert indicating a risk of at least one of oxygen deprivation, infection and sepsis.

The method may further comprise filtering the data received from the sweat sensor, using at least one of a low pass filter and a peak fitting and subtraction approach.

According to third specific aspect, there is provided an apparatus configured to stimulate sweat generation from sweat glands of a subject, the apparatus comprising: a processor configured to perform the steps of any of the methods disclosed herein.

According to a fourth specific aspect, there is provided a computer program product comprising a non-transitory computer-readable medium, the computer-readable medium having computer-readable code embodied therein, the computer-readable code being configured such that, on execution by a suitable computer or processor, the computer or processor is caused to perform steps of any of the methods disclosed herein.

These and other aspects will be apparent from and elucidated with reference to the embodiment(s) described hereinafter.

### BRIEF DESCRIPTION OF THE DRAWINGS

Exemplary embodiments will now be described, by way of example only, with reference to the following drawings, in which:
Fig. 1 is a schematic illustration of an example of a system including a sweat stimulation device;
Fig. 2 is a schematic illustration of a further example of a sweat stimulation device;
Fig. 3 is a schematic illustration of a further example of part of a sweat stimulation device;
Fig. 4 is a schematic illustration of a further example of part of a sweat stimulation device;
Fig. 5 is a schematic illustration of an example of an electrical field generated between a pair of electrodes;
Fig. 6 is a cross-sectional schematic illustration of an example of a sweat stimulation device;
Fig. 7 is a cross-sectional schematic illustration of a further example of a sweat stimulation device;
Fig. 8 is a cross-sectional schematic illustration of a further example of a sweat stimulation device;
Fig. 9 is a cross-sectional schematic illustration of a further example of a sweat stimulation device;
Fig. 10 is a graph showing an example of a sweat response over time;
Fig. 11 is a graph showing an example of a sweat response over time;
Fig. 12 is a graph showing an example of a sweat response over time;
Fig. 13 is a graph showing an example of a sweat response over time;
Fig. 14 is a flowchart of an example of a method for stimulating sweat generation;
Fig. 15 is a flowchart of a further example of a method for stimulating sweat generation;
Fig. 16 is a schematic illustration of an example of an apparatus configured to stimulate sweat generation from sweat glands of a subject; and
Fig. 17 is a schematic illustration of an example of a processor in communication with a computer readable medium.

### DETAILED DESCRIPTION OF EMBODIMENTS

Monitoring and analysis of sweat of a subject may provide an insight into the subject's health and well-being. For example, an analysis of biomarkers contained within sweat, which may contain physiologically and metabolically rich information, and may reveal information relating to a subject's health status. Biomarkers may include, among others, sodium ions (Na+), chloride ions (C1+), potassium ions (K+) and the like, which may be used to monitor dehydration. Biomarkers may also include lactate, which may be used as an early warning sign for inflammation and which, in turn, may be used to detect sepsis. Biomarkers may also include glucose, which may be used to detect, among other things, diabetes. Other types of biomarker can include cortisol, which may be used to monitor sleep (e.g. a quality of sleep) and/or stress (e.g. a level of stress) of a subject. As noted above, one known problem with measuring and analysing biomarkers in sweat is that people often fail to produce enough sweat for analysis. One way in which prior art systems have attempted to circumvent this problem is by bringing wearable sensors into nearly immediate contact with sweat as it emerges from the skin of a subject. However, sufficient amounts of sweat tend not to be produced from people if they are not performing exercise. The amount of sweat produced by a subject at ambient temperature with only light exercise or light labour is relatively low. In the so-called thermal neutral zone (e.g. in the range of 25 to 30 degrees Celsius for a naked man at rest), the core temperature may remain stable and there would therefore be no reason to induce sweat production for cooling down the body of the subject. For a subject at rest wearing clothes, the thermal neutral zone is typically relatively lower (e.g. in the range of about 13 to 22 degrees Celsius). Hence, when the temperature is in this zone and the subject is at rest, sweat production is typically almost zero. In this case (i.e. in resting and thermal neutral conditions), the sympathetic discharge (i.e. secretion of sweat by a coil of a gland) may not elicit measurable sweating since sweat reabsorption may match its formation rate.

When a person's temperature is elevated above the temperature range associated with the thermal neutral zone, the body requires cooling and indeed the sweat production rate is increased.

Monitoring the on-set of sweat production may be useful as an early warning tool and/or as a diagnostic tool. In addition, monitoring of biomarkers in sweat over time may be useful in progress monitoring in lifestyle and clinical applications. However, this can be hampered by the fact that a person in the so-called thermal neutral zone typically does not sweat and a person in a sedentary state typically has a minimal sweat rate. Existing techniques for stimulating sweat production in a subject involve applying continuous stimulation, but such stimulation may induce adaptation of the body (e.g. the subject's body becomes accustomed to the stimulation), causing an effect referred to as the "die-away effect", resulting in a reduction in sweat rate.

Embodiments of the invention described herein relates to a modulation technique for sweat stimulation, capable of inducing sweat generation in a subject, which can be used for biomarker analysis, without having to rely on exercise or systemic heating of the human body. Embodiments disclosed herein enable a limited number of sweat glands to be stimulated by restricting the stimulation to a limited skin area and localizing stimulation to those parts of the skin where sweat glands are located (typically in the lower part of the dermis). In this way, side effects such as pain by stimulating nerve endings in the skin (typically mainly in the epidermidis) may be avoided. A further advantage of an embodiment of the invention, which will be apparent from the discussion below, is the prevention or reduction in the evaporation of sweat that is generated by the disclosed stimulation techniques. Such advantages may be achieved through the control of humidity in a device that collects and transports sweat towards a sweat rate sensor and biomarker sensors. As will also become apparent from the discussion below, by modulating the stimulation applied to the subject (e.g. applying stimulation according to some modulation), the measurement of the concentration of biomarkers in the generated sweat can be used as an early warning for an upcoming hot flush and/or for the diagnosis of some diseases.

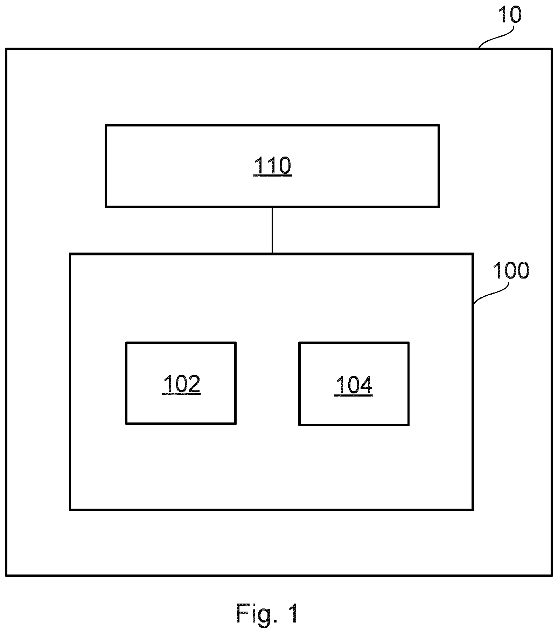

Referring now to the drawings, Fig. 1 is a schematic illustration of an example of a system 10 including a sweat stimulation device 100 for stimulating sweat generation from sweat glands of a subject and a processor 110 in communication with the sweat stimulation device. The sweat stimulation device 100 comprises a stimulation element 102 configured to provide modulated stimulation to the sweat glands through skin of the subject, and a sensor 104 for measuring data indicative of a characteristic of sweat generated by the sweat glands in response to the stimulation. The data may, for example, comprise one or more values relating to the characteristic of sweat. In some examples, the sweat stimulation device 100 may comprise a communication element (not shown) for providing the measured data to the processor 110 for processing. In some examples, the processor 110 may form part of the device 100 and data measured by the sensor 104 may be provided to the processor from the sensor by a wired or wireless connection. For example, the processing circuitry may be provided in communication (e.g. wired or wireless communication) with the stimulation element 102 and/or the sensor 104. In other examples, the processor 110 may be located remotely with respect to the sweat stimulation device 100. For example, the processor 110 may form part of a remote computing system, a remote server, a central monitoring system, a smartphone, or some other apparatus in communication with the sweat stimulation device 100. Data measured by the sensor 104 may be provided to the processor 110 from the sensor by a wired or wireless connection.

The processor 110 is configured to receive the measured data from the sensor 104; compare the measured data with a threshold value; and responsive to determining that the measured data meets or exceeds the threshold value, generate an alert signal. The processing of the data is discussed below. In general, however, the steps performed by the processor 110 enable a detection to be made of a potential medical condition from which the subject may be suffering and, therefore, the system 100 can provide an early warning system to alert a user (e.g. the subject or a medical professional) of the medical condition, based on the measurements made in relation to the subject's sweat.

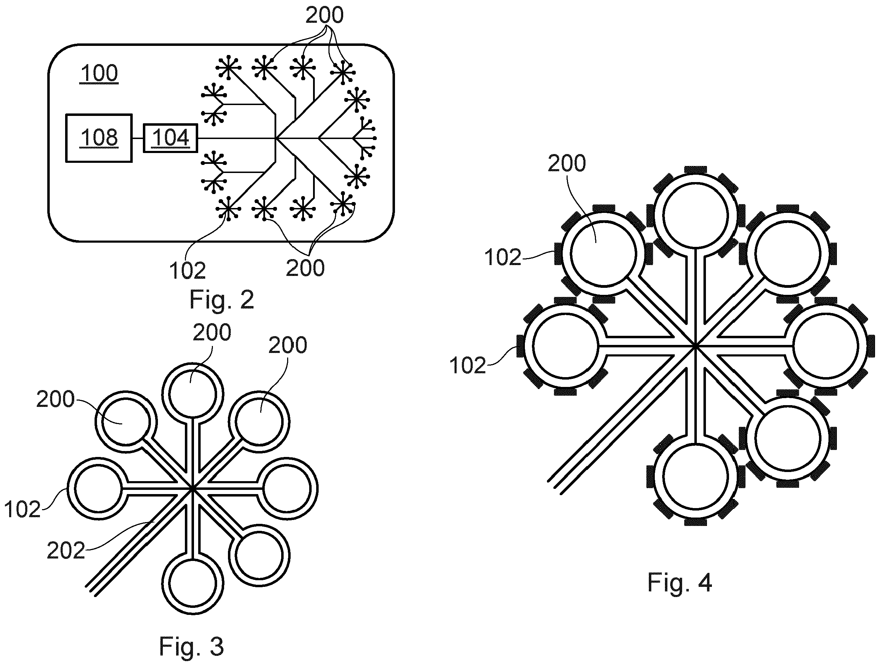

Fig. 2 is a schematic illustration of a further example of the sweat stimulation device 100 according to a specific embodiment. In the example shown in Fig. 2, the sweat stimulation device 100 is in the form of a patch that can be applied to (e.g. adhered to) the skin of the subject. In this example, sweat is collected from the subject's skin using a plurality of collection regions 200 and transported to the sensor 104. While, in this example, the collection regions form part of the sweat stimulation device 100, it will be understood that, in other examples, the collection regions may be separate from, and/or located remotely from the sweat stimulation device, and may transport collected sweat to the sensor 104 via a sweat transport system. The sweat generation device 100 may further comprise an absorber 108 configured to absorb sweat once the sweat has passed through and/or been analysed by the sensor 104. The absorber helps to prevent "used" sweat (i.e. sweat that has been analysed) from mixing with "fresh" sweat, that is yet to be analysed.

Fig. 3 is an enlarged illustration showing some collection regions 200 in greater detail. Each collection region 200 in this example is connected via one or more fluid channels 202 to the sensor 104.

In the example shown in Fig. 3, the stimulation element 102 comprises an element that extends around the perimeter of each collection region 200. In other examples, the stimulation element 102 may be integrated within one or more of the collection regions 200.

The stimulation element 102 may utilise any technique suitable for stimulating sweat generation in a subject (e.g. for stimulating a sweat gland in order to cause the sweat gland to generate sweat). According to various embodiments disclosed herein, the stimulation element 102 may comprise a heating element for stimulating the generation of sweat using heat, or an electrical element for stimulating the generation of sweat using electrical pulses.

In some embodiments, the stimulation element 102 may comprise a heating element operative to apply a modulated stimulation comprising a variation in temperature. In such embodiments, the stimulation element 102 may comprise a wire or an electrical resistor, and heat may be produced by sending an electrical current through the wire or resistor. In other examples, the stimulation element 102 may comprise a channel or passage, such as a pipe, configured to carry a heated fluid. For example, air, water or some other fluid may be heated, then pumped through the channel or, in some embodiments, the fluid may be heated as it passes through the channel. The channel may form a closed loop or circuit within the sweat stimulation device 100, such that fluid is able to flow continuously around the circuit. Thus, in some embodiments, the sweat stimulation device may comprise a pump or fan for pumping the fluid, or otherwise causing the fluid to move through the channel. The sweat stimulations device 100 may, in some embodiments, comprise a reservoir, such as a fluid reservoir, to store fluid (e.g. water, air or some other liquid) which is to flow through the channel.

The sweat stimulation device 100 may, in some embodiments comprise multiple stimulation elements 102, or the stimulation element may be formed of multiple components. Fig. 4 is an enlarged illustration of a further example of some of the collection regions 200. In this example, a plurality of stimulation elements 102 may be formed in, on or around each collection region 200. Each stimulation element 102 may, for example, comprise a heating element such as a heat source, or an electrical element, such as an electrode or a pair of electrodes. In some embodiments, the plurality of stimulation elements 102 may be configured to operate independently of one another (e.g. one or more of the stimulation elements may be configured to provide a stimulation while others do not) while, in other embodiments, all of the stimulation elements may be operated simultaneously. The sweat stimulation device 100 may thereby provide localized stimulation of a subject (e.g. stimulation of an area of skin of the subject containing a sweat gland or multiple sweat glands) using one or more of the stimulation elements 102.

In some embodiments, one or more of the stimulation elements 102 may comprise heat sources configured to generate infrared radiation. Advantageously, infrared radiation provides efficient penetration into the skin, and this may be further improved with the use of one or more optical elements, such as lenses. In embodiments where radiation is used to increase the temperature of the skin in order to stimulate sweat generation, a radiation source may be positioned in or on a housing of the sweat generation device 100. The housing may be formed from a material that is transparent to the generated radiation. In some embodiments, the light emitting diodes (LEDs) may be used to generate radiation. In a particular embodiment, one or more blue LEDs may be provided to stimulate blood perfusion, e.g. to stimulate blood flow to tissues such as the skin, and one or more infrared LEDs may be used to generate heat for stimulating sweat generation. Such a combination of radiation sources may have a lower onset temperature for inducing sweat generation.

According to some embodiments, the sweat generation device 100 may comprise a temperature sensor (not shown). Such a temperature sensor may be configured to measure temperature data indicative of a temperature of a region of the skin of the subject around the stimulation element 102 (or around one of the stimulation elements). For example, the temperature sensor may measure the temperature of skin affected by one or more of the sweat stimulation elements 102 and/or the temperature of one or more of the sweat stimulation elements themselves. In some embodiments, a measurement acquired using the temperature sensor, e.g. a temperature measurement of a heating element, the air around the heating element, the skin of the subject, or the like, may be provided as an input to a processor or controller that controls a heating level of the heating element. To prevent overheating of the skin of a subject (e.g. in the case of electrical heating), the temperature sensor may be located on the skin. In some examples, multiple temperature sensors, e.g. a first temperature sensor and a second temperature sensor, may be employed, particularly if the heat generation over the extent of the sweat stimulation device 100 is not uniform. In some examples, the one or more temperature sensors may be located on or within the sweat generation device 100 while, in other examples, the temperature sensor(s) be located remotely from the sweat generation device, and a measured temperature may be transmitted to the processor or controller that controls the heating level of the heating element. For example, in the scenario in which heated fluid is used to stimulate sweat generation, the temperature sensor(s) may be located at or around the point of heat generation (e.g. at the end of the heating element). In some examples, the fluid may be heated at a location remote from the skin of the subject and or remote from the sweat stimulation device 100. In examples where multiple temperature sensors are provided, a first temperature sensor may be positioned at or near to the centre of the sweat generation device 100 while a second temperature sensor may be positioned at or near to an outer edge of the sweat generation device. For example, a temperature sensor may be located within or adjacent to a collection region 200, between two collection regions, at a central location approximately equidistant from a plurality of collection regions, adjacent to a stimulation element 102, or the like. A controller may use a feedback mechanism (e.g. a fast feedback loop) to control the amount of heat generated by the heating element and thus the temperature of the skin based on the measured temperature(s). In examples in which a flow of heated fluid is used to stimulate sweat generation, the temperature sensor may be located at the end of the heating element. In some examples, the component used to heat the fluid may be spaced apart from the skin (i.e. not adjacent to the skin).

To achieve modulation of the stimulation in embodiments in which the stimulation element 102 comprises a heating element, a cyclic behaviour of heating and cooling may be obtained either by passively cooling the heating element by reducing the amount of heat generated (e.g. by deactivating a heating element) or by active cooling. In examples in which the heating element is an electrical wire or electrical resistor, passive cooling may be achieved by removing an electrical current passing through the resistor. Cooling of the electrical wire or resistor may be achieved through heat loss to the skin and the surrounding environment. The construction of components of the sweat generation device 100, such as the stimulation element 102, may be such that equilibrium with skin and/or the surrounding environment may be reached quickly. For example, components may be constructed so as to be thin with relatively low mass. In examples in which the heating element is a channel through which a fluid is pumped, the fluid in the reservoir may be heated and/or cooled by thermoelectric cooling, for example using a Peltier cooling element. In this case, a temperature sensor may be positioned in the reservoir.

In some embodiments, the stimulation element 102 may comprise an electrical element operative to apply a modulated stimulation comprising a variation in electrical current. In some examples, the stimulation element 102 may comprise two or more electrodes connected to or in electrical communication with the skin of a subject, and configured to conduct current pulses (e.g. in the order of a few mA) between the electrodes through the subject's skin. In some examples, the modulated stimulation may be provided using Transcutaneous Electro Neuro Stimulation (TENS) techniques.

The example of the sweat stimulation device 100 shown in Fig. 4 may implement an electrical element. For example, each of the plurality of stimulation elements 102, comprises an electrical element operative to apply a modulated stimulation comprising a variation in electrical current. In some examples, each stimulation element 102 may comprise a pair of electrodes, or one of a pair of electrodes. Modulation of stimulation provided by an electrical element may be achieved by varying an electrical current delivered or conducted through the electrical element or between two electrodes. For example, current pulses, which may be generated using a battery-operated device, may be transferred between two electrodes on the skin surface of a subject. In some examples, an AC voltage may be applied over a positive and a negative electrode resulting in an electrical field being radiated into skin of a subject and thus stimulating one or more sweat glands of the subject (e.g. an eccrine sweat gland). In a simple case, modulation of the stimulation may be achieved by starting and stopping actuation of the electrodes. The electrodes may be configured such that optimal actuation of the nerves that innervate eccrine sweat glands is achieved, while causing minimal actuation of the subject's muscles. For example, the geometric design (e.g. shape, thickness and/or relative positions) of the electrodes may be such that the intensity of the electrical pulse at a tissue depth corresponding to the relevant nerves is optimised.

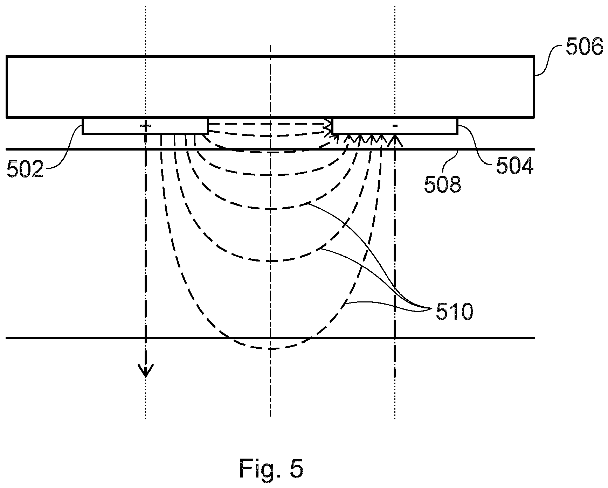

Fig. 5 is a schematic illustration of an example of an electrical field generated from a pair of electrodes, e.g. TENS electrodes. A positive electrode 502 and a negative electrode 504 are supported by an electrode support 506, and are positioned on or adjacent to a surface 508 of the subject's skin. In examples where an alternating current is applied to the electrodes 502, 504, the positive electrode 502 and negative electrode 504 may alternate between positive and negative polarities. The electrodes 502, 504 are configured to generate an electric field as represented by electric field lines 510. The depth of an electrical field (e.g. an electric field strength) into the skin may be determined by one or more of the separation between the pair of electrodes, the pitch distance between the pair of electrodes (i.e. the distance between the centres of two electrodes) and/or a voltage difference between the pair of electrodes. In some examples, the depth of the electric field may be influenced by having electrodes of different polarities on different planes.

Characteristics of the current pulses may comprise a pulse waveform (e.g. monophasic, symmetrical biphasic, asymmetrical biphasic, or the like), a pulse amplitude (e.g. 1 to 50 mA into a 1kΩ load, or the like), a pulse duration (e.g. 10 to 1000 µs), a pulse frequency (e.g. 1 to 250 Hz, or the like), and/or a pulse pattern (e.g. continuous, burst, modulated in frequency, amplitude, pulse duration or random modulation, or the like). Different nerves or classes of nerves may be stimulated by varying an electrical current between two electrodes including nerves that activate muscles, pain nerves and/or thermally sensitive nerves. Thus, parameters of the electrodes or the electric field may be selected in order to stimulate nerves associated with the subject's sweat glands.

In some embodiments, current pulses may be provided through the electrical element (e.g. between electrodes of electrode pairs) having characteristics (e.g. frequencies or intensities) selected such that nerves may be stimulated while not producing (or minimising production of) heat in tissues directly. In some cases, the current passing through the electrical element may actuate nerves that initiate muscle contractions, which may produce heat. This heat may have a systemic effect such that the hypothalamus of a subject may induce sweating to cool the body of the subject and thus may raise the temperature of neighbouring skin tissue, which may result in sweat glands in the neighbouring tissue producing sweat. In a second effect, the electrical element may actuate nerves that innervate eccrine sweat glands of the subject, which may produce sweat. In some examples, standard TENS elements may be integrated into the sweat generation device 100 in order to achieve one or more of the effects mentioned above. A frequency of a current or of current pulses passing through the electrical element may be tuned to improve an effectiveness of activating nerves responsible for innervating the sweat glands while minimizing activation of pain nerves in the skin. In some examples, an intensity and/or frequency output of a current or of current pulses passing through the electrical element may be varied (e.g. modulated). For example, raising an intensity of the electrical element (e.g. increasing the current that passes between two electrodes) may provide a relatively higher efficiency in actuating nerves innervating eccrine sweat glands.

A response to the application of an electric current to the skin or tissue of the subject may be measured using a skin conductance sensor. Thus, in some embodiments, the sweat generation device 100 may comprise a skin conductance sensor to measure a response signal indicative of skin conductance of the subject. Such a response signal may be used to adapt stimulation parameters. For example, a response signal acquired using the skin conductance sensor may be used as a control signal, or may be used to generate a control signal, to adapt stimulation parameters (e.g. amplitude, time, frequency, duty cycle, or the like). In this embodiment, stimulation of the sweat glands may be provided using an electrical element (e.g. a TENS device) or using radiofrequency electrodes attached to skin of a subject. Radiofrequency (RF) electrodes may include a thin wire configured to carry high frequency electromagnetic waves that create heat used for stimulating the sweat glands of the subject. The stimulus response signal may, in some embodiments, comprise a galvanic skin response (GSR) signal indicating skin conductance of the subject and providing an indication of the onset of sweating or sweat stimulation response. In some examples, circuity used for measuring skin conductance may also be used for providing stimulation (e.g. via a different frequency range (AC/DC) and duty cycle based voltage supply). Duty cycles may be used to switch between a skin conductance sensing (e.g. GSR) and TENS/RF simulation mode. As noted above, a control signal for controlling parameters of the stimulation (e.g. parameters of the current or current pulses) may be generated based on the response signal. In other examples, the response signal may be used to generate a control signal for controlling other components of the sweat stimulation device 100, such as the sensor 104. Once a threshold reference sweat rate/skin conductance value is reached, control electronics may activate the sensor 104 to start measuring a concentration of a biomarker in the sweat (e.g. a semi-continuous measurement).

It has been recognised that, if stimulation of sweat glands is provided to a subject continuously, for a prolonged period, then the subject's body may adapt to the stimulation as a result of a phenomenon referred to as habituation. Habituation may be defined as a decrease in a response to a repeated stimulus. In the example of electrical stimulation (e.g. using a TENS device), a sensation of tingling or buzzing in the subject's body beneath electrodes may gradually decline over time, even though the output of the sweat stimulation device 100 remains the same. In some embodiments, prevention or delay of habituation to sweat stimulation by heat stimulation and/or electrical stimulation may be achieved by modulation of the stimulation, for example by modulation of stimulus parameters. By varying one or more parameters of the stimulation, for example, phase duration, amplitude (e.g. strength), phase amplitude, pulse rate, frequency, or by varying a combination of multiple parameters, the subject's body is less likely to adapt to the stimulation, and habituation may be reduced or even prevented. Reducing the effects of habituation is advantageous as it enables long term sustainable sweat stimulation over the lifetime of use of a sweat stimulation device 100.

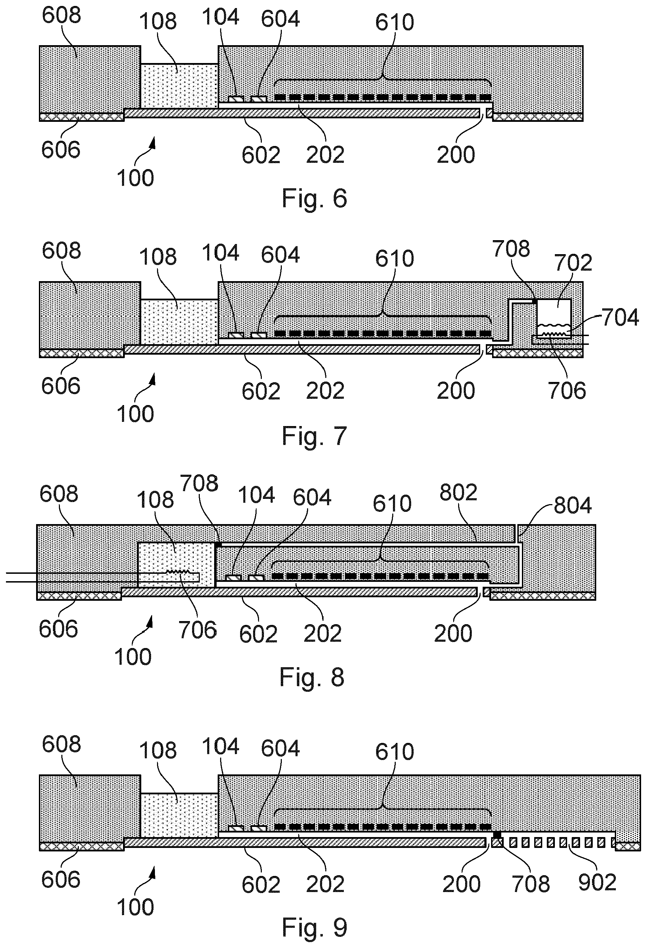

Figs. 6 to 9 are cross-sectional schematic illustrations of various examples of the sweat stimulation device 100, which is intended to be positioned on a subject's skin during use. A single collection region 200 is shown, and is depicted schematically as a perpendicular channel, but it will be understood that the sweat stimulation device 100 may include multiple collection regions 200, as shown, for example, in Figs. 2, 3 and 4. In the examples shown in Figs. 6 to 9, the sweat generation device 100 may comprise a sweat collection plate 602 which, together with a housing 608, forms a fluidic channel 202 for transporting the collected sweat through the sweat stimulation device, a sweat rate sensor 604 for measuring a sweat rate of sweat glands of the subject, one or more sensors 104 (e.g. biosensors for measuring or determining biomarker concentrations, sweat rate sensors or sensors to measure some other characteristic) for analysing sweat, for example by measuring a concentration of a biomarker of the sweat, an absorber 108 for absorbing sweat after it has passed through the sensor(s) 104, an attachment layer 606 for aiding attachment of the sweat stimulation device to the skin of the subject, the housing 608 for housing one or more components of the sweat stimulation device and/or one or more electrowetting electrodes 610 for aiding the transport of sweat along the fluidic channel 202. The sweat rate sensor 604 may be considered to be an example of one of the sensors 104, which measures a characteristic of sweat, namely the sweat rate.

In the example shown in Fig. 6, the sweat stimulation device 100 comprises a semi-closed fluidic channel 202 (e.g. closed at one end) to restrict the amount of sweat able to evaporate and leave the sweat stimulation device. In the example shown in Fig. 7, evaporation of sweat is produced by maintaining a high level of humidity within the sweat stimulation device 100 and, particularly within the fluidic channel 202. In this example, the sweat generation device 100 comprises a fluid reservoir 702 containing fluid 704 which can be heated by a heater 706. The fluid reservoir 702 is in fluidic communication with the fluidic channel 202, but a hydrophobic filter 708 is configured to restrict or prevent the passage of liquid (e.g. water) from the reservoir into the fluidic channel 202, but to allow the passage of gas (e.g. water vapour). The heater 706 may be controlled to heat the fluid 704 in the reservoir 702, and heated fluid vapour may pass into the fluidic channel 202, thereby increasing the humidity in the fluidic channel. By increasing the humidity, evaporation of the sweat from the sweat generation device 100 is reduced.

In the example shown in Fig. 8, a humidity in the fluidic channel 202 may be increased by evaporating waste/used sweat collected by the absorber 108. In this example, after sweat has passed through the sensor 104, it is collected in the absorber 108. A heater 706 is configured to heat the sweat contained within the absorber 108 to generate fluid vapours, which are able to travel along a channel 802 and into the fluidic channel 202. A hydrophobic filter 708 prevents the passage of liquid sweat from the absorber 108 into the channel 802. A pressure release channel 804 prevents a build-up of pressure within the channel 802.

In the example shown in Fig. 9, the sweat collection plate 602 is configured to cover additional sweat glands. Apertures 902 formed in the sweat collection plate 602 allow the passage of sweat vapours generated by the additional sweat glands into the sweat generation device 100, and sweat vapours may pass into the fluidic channel 202, hence increasing the humidity in the fluidic channel, and thereby reducing the evaporation of sweat. A hydrophobic filter 708 may be utilized to prevent unintended liquid originating from apertures 902 from entering the fluidic channels 202.

In each of the examples shown in Figs. 6 to 9, sweat may be transported towards the one or more sensors 104 using electrowetting techniques (i.e. using the one or more electrowetting electrodes) or the like. In other examples, other methods of transporting sweat towards the sensors 104 may be employed.

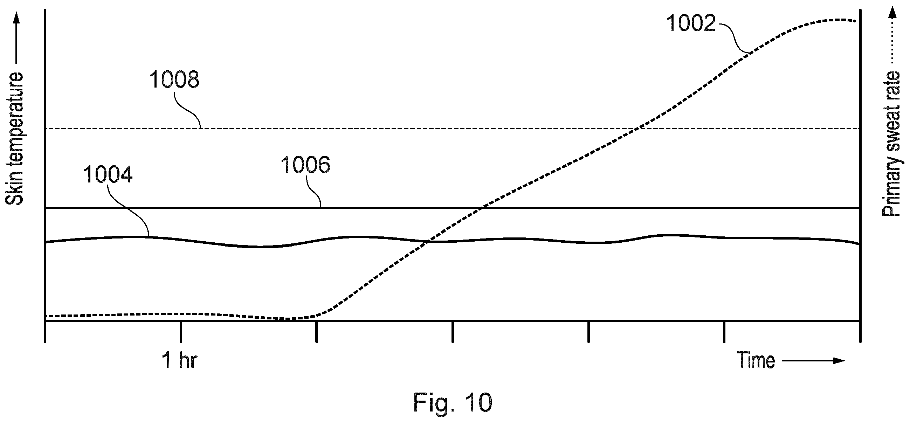

A particular advantage of embodiments disclosed herein is that sweat detected and/or analysed using the sensor 104 of the sweat generation device 100 may provide an early warning of a medical condition of the subject, as discussed below with reference to graphs shown in Figs. 10, 11, 12 and 13.

If a constant heat is applied to a subject in order to stimulate sweat generation, then the subject may start sweating due to the heat stimulation, irrespective of whether or not the subject has an underlying health condition. If heat is applied to the subject for a prolonged period of time, the subject may adapt to the heat stimulus and, subsequently, the subject's response to the stimulus may reduce or stop altogether. Thus, even if the subject is suffering from a health condition or health disorder (e.g. an infection, sepsis, or the like), adaptation of the subject's body may result in a reduction of sweat generation, and this could lead to false results (e.g. a failure to detect an underlying health condition such as an infection). Adaptation of a human body to heat occurs after around 50 minutes. Therefore, to reduce the chance that the subject adapts to the stimulation by the application of heat, a period of stimulation (e.g. a period of heat application) may be limited to between around 2 and 40 minutes, and more preferably between around 5 and 10 minutes. In some embodiments, passive or active cooling of the subject's skin may be applied for a period of between around 2 and 20 minutes, and more preferably for a period of between around 2 and 10 minutes between applications of heat.

The graphs shown in Figs. 10 to 13 demonstrate how a sweat rate of a subject, and a skin temperature of the subject, change over time in particular circumstances. The sweat rate of the subject, which may be measured, for example using a sensor 104, such as the sweat rate sensor 604, is shown by a dashed line 1002 and the skin temperature of the subject is shown by a solid line 1004. A solid horizontal line 1006 represents a thermal neutral zone threshold; for a skin temperature below a temperature corresponding to the thermal neutral zone threshold, the core temperature of the subject remains stable and there is no reason for the body to induce sweat production to cool down the body, whereas for a temperature above the thermal neutral zone threshold 1006, sweat production of the subject may be induced in order to lower the core temperature of the body. A dashed horizontal line 1008 represents a sweat rate threshold level. When the subject sweats at a rate above this threshold, then sweat will appear on the surface of their skin. If the subject's sweat rate is below this threshold, sweat will not appear on their skin due to, for example, resorption of sweat.

Fig. 10 represents a case where no heat stimulation (e.g. using the sweat stimulation device 100) is applied and a disorder (e.g. a health disorder of a subject, such as an infection, that causes the subject to sweat) induces an increase in sweat rate (represented by the dashed line 1002). When the sweat rate meets or exceeds threshold level 1008, sweat may start to appear on the skin of the subject. Thus, in this example, the subject may become aware of a medical condition only when sweat begins to appear on their skin, and there may be no early warning sign of a health disorder.

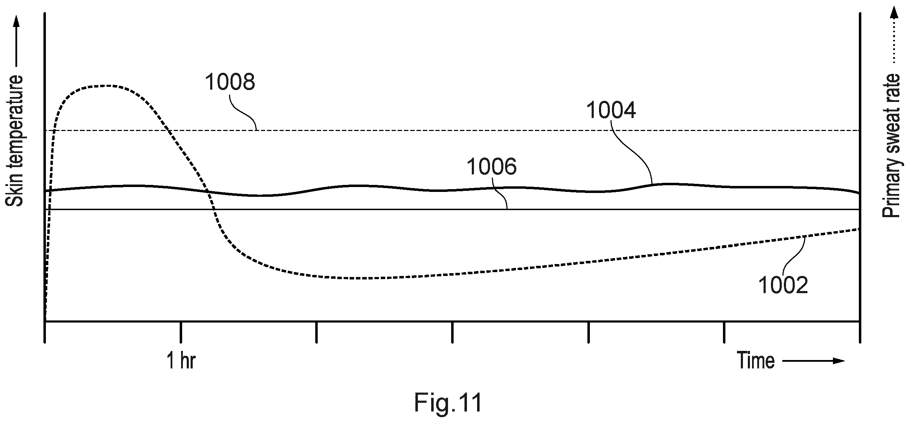

Fig. 11 represents a case where local heat stimulation (e.g. using the sweat stimulation device 100) is applied (starting at time zero) and after about for example 40 to 60 minutes, adaptation of the subject's body commences and the sweat rate decreases. In this case, if a health disorder begins causing the subject to generate sweat (e.g. after around 2 hours), the sweat rate may increase slowly, and may not increase sufficiently for sweat to appear on the subject's skin. Thus, due to the prolonged application of heat, the subject may not become aware of the medical disorder through sweat detection.

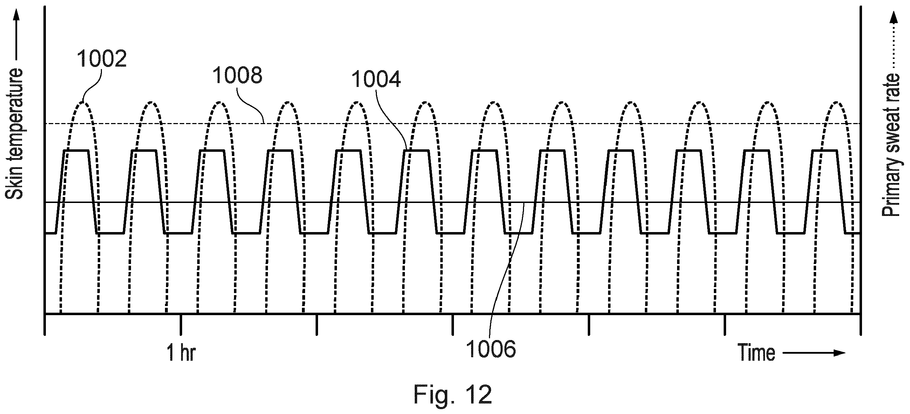

By applying the stimulation in a modulated fashion, as disclosed herein, for example by applying a cycle of heating and cooling, the sweat generation device 100 may reduce the amount by which the subject's body adapts to the stimulation. Furthermore, applying heat in a cyclic manner results in a more pleasant experience for the subject than applying heat continuously. Moreover, cyclic heating requires less power than continuous heating, so the sweat generation device 100 operates under a lower energy expenditure than if continuous heating were applied. A further advantage, as is apparent from Fig. 12 is that, if the subject is not suffering from a medical disorder, then relatively small amounts of sweat are produced when local stimulation is provided using the sweat stimulation device 100 and, therefore, there is a significantly reduced chance of misinterpreting measurements as a potential medical disorder.

Fig. 12 represents a case where a stimulation element (e.g. a heating element) is used to increase and decrease in temperature (line 1004), causing a modulated heating effect in the subject's skin (i.e. causing the subject's skin to increase and decrease in temperature). Due to the modulated stimulation, no adaptation to the heat stimulation occurs; the sweat rate (dashed line 1002) of the subject increases and decreases in line with the subject's changing skin temperature. Small amounts of sweat appear regularly on the skin as the sweat rate exceeds the threshold 1008, indicating no medical disorder.

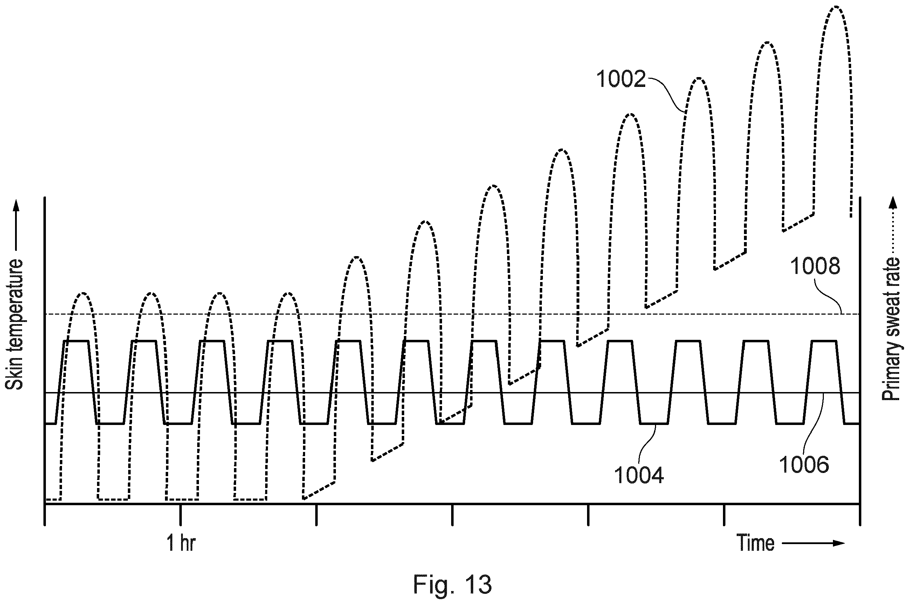

Fig. 13 similarly represents a case where a stimulation element (e.g. a heating element) is used to increase and decrease in temperature (line 1004), causing a modulated heating effect in the subject's skin (i.e. causing the subject's skin to increase and decrease in temperature). In this case, the subject suffers from a health disorder, which causes the subject to increase the rate of sweating e.g. at around two hours. It is possible to detect the moment that the amount of sweat appearing on the subject's skin starts to increase to a level beyond that associated with the small amounts of sweat produced with no medical disorder. This may serve as an early warning of a potential medical condition or disorder. For example, upon detecting an increase in the amount of sweat appearing on the subject's skin, it may be determined that some factor other than the modulated heating is causing the increased sweat rate, and further investigations (e.g. medical tests) may be made. Precise timing of detection of the increased sweat rate may depend on the particular disorder causing the increased sweating.

By employing modulated stimulation by the cyclic application of heat, the onset of increasing sweat production (i.e. due to a medical disorder) may be amplified and observed at an earlier stage than when no heat stimulation is applied.

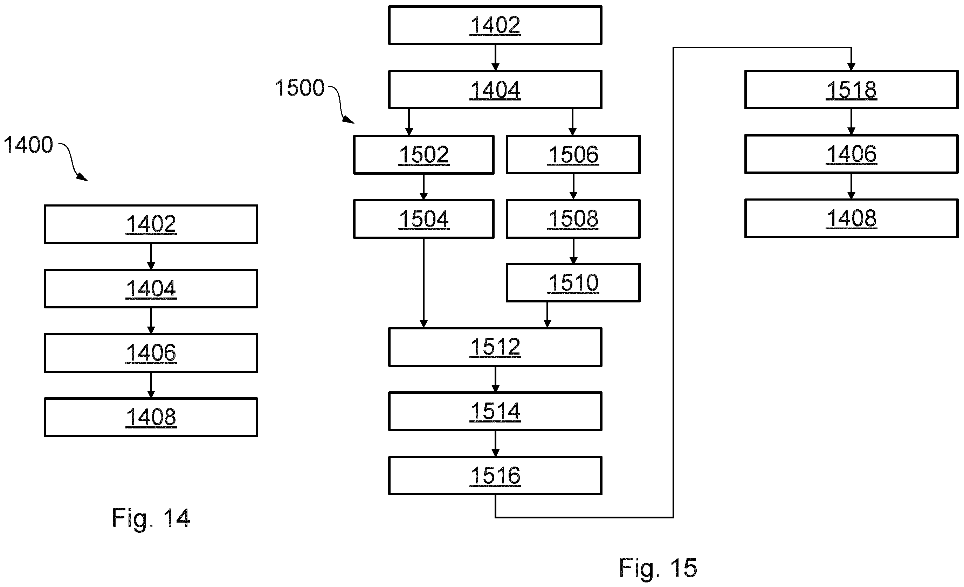

So far, the invention has been described in terms of an apparatus (e.g. a sweat generation device 100) used to provide modulated stimulation to a subject in order to cause sweat glands of the subject to generate increased levels of sweat. However, a further aspect of the present invention relates to a method. Fig. 14 is a flowchart of an example of a method 1400 of stimulating sweat generation from sweat glands of a subject. In some embodiments, the method 1400 may comprise a computer-implemented method. The method 1400 comprises, at step 1402, operating a stimulation element 102 to provide modulated stimulation to the sweat glands through skin of the subject, the modulated stimulation comprising at least one period of increased stimulation and at least one period of reduced stimulation. For example, the period of increased stimulation may comprise a period of increased temperature and/or a period of electric current or electric pulses having increased amplitude. The period of reduced stimulation may comprise a period during which the temperature and/or electric current or pulse amplitude is reduced.

At step 1404, the method 1400 comprises receiving, from a sweat sensor 104, data indicative of a characteristic of sweat generated by the sweat glands in response to the stimulation. In some embodiments, the characteristic of sweat may comprise an amount of sweat (e.g. a volume) while, in other embodiments, some other characteristic may be measured, such as a rate of sweat production/secretion (e.g. the sweat rate) or a concentration of a biomarker present in the sweat. The method 1400 comprises, at step 1406, comparing the received data with a threshold value. At 1408, the method 1400 comprises, responsive to determining that the received data meets or exceeds the threshold value, generating an alert signal. For example, if a value of the received data meets or exceeds the defined threshold value, then it may be determined that the data is indicative of a potential medical disorder or condition requiring further attention or investigation. Thus, the alert signal may comprise a signal to alert the subject of the potential concern or to alert a medical professional of the same. In one example, the modulated stimulation of the sweat glands may cause the sweat glands to generate sufficient amounts of sweat to appear at the surface of the subject's skin and, therefore, to be detected by the sensor. The modulation of the stimulation causes repetitive peaks in the amount of sweat generated (as shown, for example, in Fig. 12) and, if the amount of sweat generated during the application of stimulation exceeds a threshold, then this may be a sign of a potential medical disorder. Thus, the method may be used to provide an early warning of such medical disorders which cause sweating.

Fig. 15 is a flowchart of a further example of a method 1500 (e.g. a computer-implemented method) of stimulating sweat generation from sweat glands of a subject, which may include steps of the method 1400 discussed above.

As discussed herein, the stimulation element 102 may, in some embodiments, comprise a heating element. In such embodiments, the modulated stimulation may comprise a variation in temperature of the heating element, such that the at least one period of increased stimulation comprises an increase in temperature of the heating element and the at least one period of reduced stimulation comprises a decrease in temperature of the heating element. Passive or active cooling may be used to bring about the decrease in temperature of the heating element and/or the subject. In examples where a heating element is used to provide the modulated stimulation, the method 1500 may further comprise, at step 1502, receiving, from a temperature sensor, temperature data indicative of a temperature of a region of the skin of the subject around the heating element. At step 1504, the method 1500 may comprise adjusting an operating parameter of the heating element based on the received temperature data. In this way, a feedback loop may be used to prevent a temperature of the subject's skin from exceeding a threshold temperature (e.g. a temperature that might be considered uncomfortable for the subject). In response to the measured temperature exceeding the defined threshold, the output of the heating element may be reduced.

In other embodiments, the stimulation element 102 may comprise an electrical element operative to conduct an electrical current through the skin. In such embodiments, the modulated stimulation may comprise a series of electrical pulses, such that the at least one period of increased stimulation comprises an increase in electrical current and the at least one period of reduced stimulation comprises a decrease in electrical current. In examples where an electrical element is used to provide the modulated stimulation, the method 1500 may comprise, at step 1506, receiving, from a skin conductance sensor, a response signal indicative of skin conductance of the subject. At step 1508, the method 1500 may comprise adjusting an operating parameter of the electrical element based on the received stimulus response signal. In this way, a feedback loop may be used to prevent the subject from experiencing an electrical current exceeding a defined threshold. Alternatively, if it is determined that the modulated stimulation using the electrical element is causing excessive amounts of sweat to be generated, then an operating parameter of the electrical element may be adjusted to reduce the levels of sweat being generated. In some embodiments, the response signal received from the skin conductance sensor at step 1506 may be used to determine when the sweat sensor 104 should be activated. Thus, at step 1510, the method 1500 may comprise, responsive to determining that the received response signal meets a defined condition, activating the sweat sensor 104.

In order to reduce or prevent habituation (e.g. adaptation of the subject's body to the stimulation), some embodiments of the invention involve modifying or varying one or more parameters of the modulated stimulation. Thus, the method 1500 may further comprise, at step 1512, operating the stimulation element 102 to modify, during the at least one period of increased stimulation, at least one of a duration, an amplitude, a frequency and a duty cycle of the modulated stimulation.

An example of one biomarker that may be detected in sweat, and whose concentration in sweat may be measured, is lactate. The concentration of lactate in sweat may be indicative of an amount of oxygen in the blood of the subject. Oxygen deprivation can be an indication of an infection, and upcoming infection and/or sepsis in a subject. In a subject where there is no oxygen deprivation, the lactate concentration is likely to be relatively low, with small contributions from both the blood and the sweat glands (the latter because the subject - likely a patient - is in a sedentary state and will not sweat much). However, the moment there is oxygen deprivation in the subject, the lactate concentration in blood may start to rise. This would also lead to an increase in lactate concentration in sweat as well: (i) as originating from blood, but also (ii) the lactate originating from sweat gland cells themselves due to the lack of oxygen. On the other hand, there is a counter effect: blood is directed mainly to a subject's internal organs during an oxygen deprivation event and the blood supply to the skin reduces significantly. Hence, the supply of nutrients (e.g. glucose) to the sweat glands reduces and this may result in the sweat glands doing less work and therefore producing lower levels of lactate and smaller amounts of sweat. Consequently, these counteractive phenomena prevent a clear relationship between the lactate concentration in sweat and oxygen deprivation.

Using embodiments of the present invention however, modulated sweat stimulation is used to induce sweat generation, so even a subject in a sedentary state is stimulated to sweat. Stimulation using either a heating element or an electrical element will promote blood flow to the sweat glands and, therefore, the counter effect discussed above is reduced, restoring (or improving) the relationship between the lactate concentration in sweat and oxygen deprivation.

Thus, in some embodiments, the method 1500 may comprise, at step 1514, determining, using the sweat sensor 104 (i.e. a biosensor), an indication of an amount of lactate generated by the sweat glands. At step 1516, the method 1500 may comprise comparing the determined amount of lactate to a lactate threshold value. Responsive to determining that the amount of lactate meets or exceeds the lactate threshold value, the alert signal (generated at step 1408) may comprise an alert indicating a risk of at least one of oxygen deprivation, infection and sepsis. Thus, the method may also serve as an early warning tool for upcoming oxygen deprivation.

According to some embodiments, data acquired using the sweat sensor may be filtered in order to remove data corresponding to sweat generation caused by stress and/or anxiety. Various causes of sweating can occur simultaneously, and sweating associated with stress and anxiety may complicate the assessment of sweat, for example detecting the onset of an infection. It is known that sweating related to stress and anxiety (and indeed also to that from acute factors such as acute pain) may have a very different temporal behaviour to those associated with medical conditions, such as the onset of an infection. Specifically, sweat caused by stress and anxiety typically occurs in a series of rapid peaks, with rise times of a few seconds followed by delays in the order of tens of seconds, whilst other factors may induce a slow change in the background stress level (as is indicated in the dashed lines of Figs. 10 to 13, with time constants in the order of minutes). In order to avoid such confounding factors, a low pass filter (such as a running average) may be applied (with time constants of, for example, one minute or the like), a peak fitting and subtraction approach to extract the background sweat level from the acute sweat peaks may be performed and/or sweat measurements may be discounted if there are too many rapid sweat peaks. Thus, the method 1500 may comprise, at step 1518, filtering the data received from the sweat sensor 104, using at least one of a low pass filter and a peak fitting and subtraction approach.

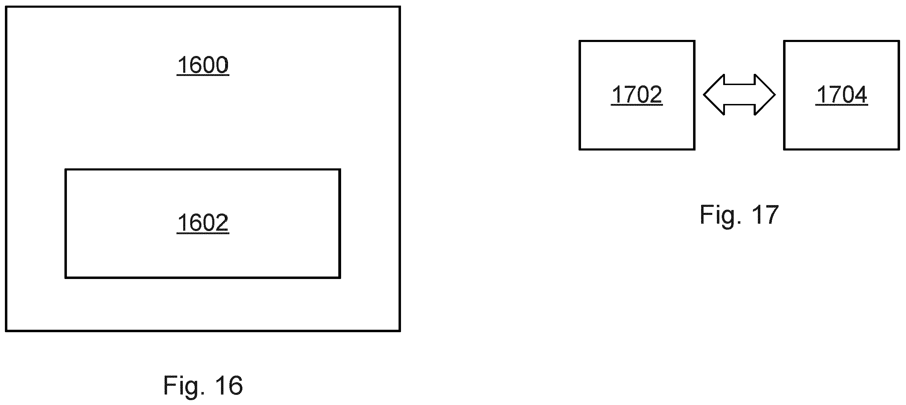

One or more of the steps of the methods 1400, 1500 disclosed herein may be performed using a processor of a computing device, such as a desktop computer, a laptop computer, a tablet computer, a smart phone, a server or processing circuitry of a cloud-computing environment. A further aspect of the present invention may, therefore, relate to an apparatus comprising such a processor. Fig. 16 is a schematic illustration of an example of an apparatus 1600 configured to stimulate sweat generation from sweat glands of a subject. The apparatus 1600 comprises a processor 1602 configured to perform one or more steps of the methods 1400, 1500 disclosed herein.

A further aspect of the present invention relates to a computer program product. Fig. 17 is a schematic illustration of an example of a processor 1702 in communication with a computer readable medium 1704. According to various embodiments, a computer program product comprises a non-transitory computer-readable medium 1704, the computer-readable medium having computer-readable code embodied therein, the computer-readable code being configured such that, on execution by a suitable computer or processor 1702, the computer or processor is caused to perform one or more steps of the methods 1400, 1500 disclosed herein.

The occurrence of sweating can have several causes, such as hot flushes, or night sweats related to infections (e.g. Lyme disease, HIV, etc.), sleep problems (OSA, etc.) or stress and/or anxiety. Aspects of the present invention disclosed herein may aid the diagnosis and monitoring of a medical disorder such as those discussed herein. A change in sweat characteristics may indicate a change in a disorder and may be an early warning for upcoming serious complications. It may support the prediction of an upcoming exacerbation of a COPD patient, so the patient can take measures to prevent or minimize the exacerbation.

The processor 1602, 1702 can comprise one or more processors, processing units, multi-core processors or modules that are configured or programmed to control the apparatus 100, 1600 in the manner described herein. In particular implementations, the processor 1602, 1702 can comprise a plurality of software and/or hardware modules that are each configured to perform, or are for performing, individual or multiple steps of the method described herein.

The term "module", as used herein is intended to include a hardware component, such as a processor or a component of a processor configured to perform a particular function, or a software component, such as a set of instruction data that has a particular function when executed by a processor.

It will be appreciated that the embodiments of the invention also apply to computer programs, particularly computer programs on or in a carrier, adapted to put the invention into practice. The program may be in the form of a source code, an object code, a code intermediate source and an object code such as in a partially compiled form, or in any other form suitable for use in the implementation of the method according to embodiments of the invention. It will also be appreciated that such a program may have many different architectural designs. For example, a program code implementing the functionality of the method or system according to the invention may be sub-divided into one or more sub-routines. Many different ways of distributing the functionality among these sub-routines will be apparent to the skilled person. The sub-routines may be stored together in one executable file to form a self-contained program. Such an executable file may comprise computer-executable instructions, for example, processor instructions and/or interpreter instructions (e.g. Java interpreter instructions). Alternatively, one or more or all of the sub-routines may be stored in at least one external library file and linked with a main program either statically or dynamically, e.g. at run-time. The main program contains at least one call to at least one of the sub-routines. The sub-routines may also comprise function calls to each other. An embodiment relating to a computer program product comprises computer-executable instructions corresponding to each processing stage of at least one of the methods set forth herein. These instructions may be sub-divided into sub-routines and/or stored in one or more files that may be linked statically or dynamically. Another embodiment relating to a computer program product comprises computer-executable instructions corresponding to each means of at least one of the systems and/or products set forth herein. These instructions may be sub-divided into sub-routines and/or stored in one or more files that may be linked statically or dynamically.

The carrier of a computer program may be any entity or device capable of carrying the program. For example, the carrier may include a data storage, such as a ROM, for example, a CD ROM or a semiconductor ROM, or a magnetic recording medium, for example, a hard disk. Furthermore, the carrier may be a transmissible carrier such as an electric or optical signal, which may be conveyed via electric or optical cable or by radio or other means. When the program is embodied in such a signal, the carrier may be constituted by such a cable or other device or means. Alternatively, the carrier may be an integrated circuit in which the program is embedded, the integrated circuit being adapted to perform, or used in the performance of, the relevant method.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A system (10) comprising:
a sweat stimulation device (100) for stimulating sweat generation from sweat glands of a subject, the device comprising:
a stimulation element (102) configured to provide modulated stimulation to the sweat glands through the skin of the subject; and
a sensor (104) for measuring data indicative of a characteristic of sweat generated by the sweat glands in response to the stimulation; and
a processor (110) configured to:
receive the measured data from the sensor;
compare the measured data with a threshold value; and
responsive to determining that the measured data meets or exceeds the threshold value, generate an alert signal.

2. A system (10) according to claim 1, wherein the stimulation element (102) comprises at least one of: a heating element operative to apply a modulated stimulation comprising a variation in temperature; and an electrical element operative to apply a modulated stimulation comprising a variation in electrical current.

3. A system (10) according to claim 1 or claim 2, wherein the sweat stimulation device (100) further comprises:
a channel (202) configured to transport sweat from skin of the subject to the sensor (104);
a reservoir (702) for containing a fluid, the reservoir in fluidic communication with the channel; and
a reservoir heater (706) to heat fluid contained within the reservoir, thereby to increase the humidity in the channel.

4. A system (10) according to any of claims 1 to 3, wherein the sweat stimulation device (100) further comprises at least one of: a temperature sensor to measure temperature data indicative of a temperature of a region of the skin of the subject around the stimulation element (102); and a skin conductance sensor to measure a response signal indicative of skin conductance of the subject.

5. A method (1400) of stimulating sweat generation from sweat glands of a subject, the method comprising:
operating (1402) a stimulation element (102) to provide modulated stimulation to the sweat glands through skin of the subject, the modulated stimulation comprising at least one period of increased stimulation and at least one period of reduced stimulation;
receiving (1404), from a sweat sensor (104), data indicative of a characteristic of sweat generated by the sweat glands in response to the stimulation;
comparing (1406) the received data with a threshold value; and
responsive (1408) to determining that the received data meets or exceeds the threshold value, generating an alert signal.

6. A method (1400) according to claim 5, wherein the stimulation element (102) comprises a heating element; and
wherein the modulated stimulation comprises a variation in temperature of the heating element, such that the at least one period of increased stimulation comprises an increase in temperature of the heating element and the at least one period of reduced stimulation comprises a decrease in temperature of the heating element.

7. A method (1400, 1500) according to claim 6, further comprising:
receiving (1502), from a temperature sensor, temperature data indicative of a temperature of a region of the skin of the subject around the heating element; and
adjusting (1504) an operating parameter of the heating element based on the received temperature data.

8. A method (1400, 1500) according to claim 5, wherein the stimulation element (102) comprises an electrical element operative to conduct an electrical current through the skin; and
wherein the modulated stimulation comprises a series of electrical pulses, such that the at least one period of increased stimulation comprises an increase in electrical current and the at least one period of reduced stimulation comprises a decrease in electrical current.

9. A method (1400, 1500) according to claim 8, further comprising:
receiving (1506), from a skin conductance sensor, a response signal indicative of skin conductance of the subject; and
adjusting (1508) an operating parameter of the electrical element based on the received stimulus response signal.

10. A method (1400, 1500) according to claim 8 or claim 9, further comprising:
receiving (1506), from a skin conductance sensor, a response signal indicative of skin conductance of the subject; and
responsive to determining that the received response signal meets a defined condition, activating (1510) the sweat sensor (104).

11. A method (1400, 1500) according to any of claims 5 to 10, further comprising:
operating (1512) the stimulation element (102) to modify, during the at least one period of increased stimulation, at least one of a duration, an amplitude, a frequency and a duty cycle of the modulated stimulation.

12. A method (1400, 1500) according to any of claims 5 to 11, further comprising:
determining (1514), using the sweat sensor (104), an indication of an amount of lactate generated by the sweat glands; and
comparing (1516) the determined amount of lactate to a lactate threshold value;
wherein, responsive to determining that the amount of lactate meets or exceeds the lactate threshold value, the alert signal comprises an alert indicating a risk of at least one of oxygen deprivation, infection and sepsis.

13. A method (1400, 1500) according to any of claims 5 to 12, further comprising:
filtering (1518) the data received from the sweat sensor (104), using at least one of a low pass filter and a peak fitting and subtraction approach.

14. An apparatus (1600) configured to stimulate sweat generation from sweat glands of a subject, the apparatus comprising:
a processor (1602) configured to perform the steps of the method of any of claims 5 to 13.

15. A computer program product comprising a non-transitory computer-readable medium (1704), the computer-readable medium having computer-readable code embodied therein, the computer-readable code being configured such that, on execution by a suitable computer or processor (1702), the computer or processor is caused to perform the method of any of claims 5 to 13.
